# FASCICULE DE BREVET EUROPEEN

(11) **EP 1 262 163 B1**
(45) Date de publication et mention de la délivrance du brevet: **15.08.2007**
(21) Numéro de dépôt: 02290927.9
(22) Date de dépôt: 12.04.2002
(51) Int. Cl.: A61K 8/81, A61Q 1/06

(54) **Composition à phase grasse liquide gélifée par un polymère semi-cristallin**
Zusammensetzung mit flüssiger fetter Phase, gelifiert durch ein teilkristallines Polymer
Composition having a liquid fatty phase gelified by a semicrystalline polymer

(30) Priorité: 04.05.2001 FR 0106047
(43) Date de publication de la demande: 04.12.2002
(73) Titulaire: L'ORÉAL, 75008 Paris (FR)
(72) Inventeur: Mondet, Jean, 93600 Aulnay Sous Bois (FR); Ferrari, Véronique, 94700 Maisons-Alfort (FR)
(74) Mandataire: Le Coupanec, Pascale A.M.P.

(56) Documents cités:
- EP-A- 1 034 776
- WO-A-01/19333
- US-A- 5 302 380
- US-A- 5 519 063
- US-A- 5 736 125

## Description

La présente invention se rapporte à une composition de soin et/ou de traitement et/ou de maquillage de la peau, y compris du cuir chevelu, et/ou des lèvres du visage des êtres humains, contenant une phase grasse liquide structurée par au moins un polymère particulier et se présentant notamment sous forme d'un stick de rouge à lèvres, dont l'application conduit à un dépôt brillant, couvrant et non collant.

Dans les produits cosmétiques ou dermatologiques, il est courant de trouver une phase grasse liquide structurée, à savoir rigidifiée ; ceci est notamment le cas dans les compositions solides comme les déodorants, les baumes et les rouges à lèvres, les produits anti-cerne et les fonds de teint coulés.

Par "phase grasse liquide", au sens de l'invention, on entend une phase grasse liquide à température ambiante (25°C) et pression atmosphérique (760 mm de Hg), composée d'un ou plusieurs corps gras liquides à température ambiante, appelés aussi huiles, compatibles entre eux. Cette phase grasse est macroscopiquement homogène.

La structuration de la phase grasse liquide permet en particulier de limiter son exsudation des compositions solides et, en plus, de limiter, après dépôt sur la peau ou les lèvres, sa migration au cours du temps dans les rides et ridules, ce qui est particulièrement recherché pour un rouge à lèvres. Par "migration", on entend un débordement de la composition et en particulier de la couleur, hors du tracé initial. Or, une migration importante de la phase grasse liquide, chargée de matières colorantes, conduit à un effet inesthétique autour des lèvres, accentuant particulièrement les rides et les ridules. Cette migration est souvent citée par les femmes comme un défaut majeur des rouges à lèvres classiques.

Cette structuration peut être obtenue à l'aide de particules solides ou charges. De plus, les charges permettent de réduire le toucher collant de certaines huiles comme l'huile de ricin ou les poly-isobutènes, généralement utilisés dans les rouges à lèvres.

Malheureusement, ces particules ou charges ont tendance à matifier la composition, ce qui n'est pas toujours souhaitable en particulier pour un rouge à lèvres ; en effet, les femmes sont toujours à la recherche d'un rouge à lèvres sous forme de bâton déposant un film de plus en plus brillant.

La brillance est liée pour l'essentiel à la nature de la phase grasse liquide. Ainsi, il est possible de diminuer le taux de charges de la composition pour augmenter la brillance d'un rouge à lèvres mais alors, la migration de la phase grasse liquide et/ou le toucher collant de la composition augmentent. Autrement dit, le taux de charges nécessaire à la réalisation d'un stick cosmétiquement acceptable est un frein à la brillance du dépôt. Par ailleurs, les charges ont tendance à dessécher la peau, marquer le relief cutané, en particulier les pores et rides, accentuant ainsi les imperfections locales, ce qui est contraire à l'effet recherché par les femmes en particulier pour un fond de teint aussi bien du visage que du corps humain.

Le demandeur a donc envisagé la fabrication d'un stick, comportant peu ou pas de charges, telles que celles classiquement utilisées en cosmétique. Les charges généralement utilisées dans les domaines cosmétiques et dermatologiques sont les silices, les talcs, les argiles, les kaolins, les poudres de polyamide (Nylon®).

Comme autre agent de structuration utilisé dans les domaines cosmétiques ou dermatologiques, on peut citer les cires. Les cires utilisées jusqu'à présent ont aussi tendance à matifier la composition du fait de leur structure cristalline particulière. Par ailleurs, elles confèrent à la composition un toucher généralement désagréable.

L'invention a justement pour objet une composition de soin et/ou de maquillage et/ou de traitement des matières kératiniques comme la peau et/ou les lèvres du visage et/ou les phanères permettant de remédier à ces inconvénients.

De façon surprenante, le demandeur a trouvé que l'utilisation de polymères particuliers permettait de structurer, même en l'absence de cire classique et de charge, les phases grasses liquides sous forme de stick dont l'application sur les lèvres conduisait à un film brillant, non collant, de bonne couvrance et non migrant.

L'invention s'applique non seulement aux produits de maquillage des lèvres mais aussi aux produits de soin et/ou de traitement de la peau, y compris du cuir chevelu, et des lèvres, comme les crèmes de soin journalier, les baumes à lèvres, les produits de coloration de la peau, et de protection solaire de la peau du visage, aux produits de maquillage de la peau, aussi bien du visage que du corps humain, comme les fonds de teints notamment coulés en stick ou en coupelle, les produits anti-cerne et les produits de tatouage éphémère, aux produits d'hygiène corporelle comme les déodorants notamment en stick, et aux produits de maquillage des yeux comme les eye-liners en particulier sous forme de crayon et les mascaras notamment sous forme de pain

De façon plus précise, l'invention a pour objet une composition selon la revendication 1.

La composition de l'invention peut se présenter sous forme de pâte, de solide, de crème. Elle peut être une émulsion huile-dans-eau ou eau-dans-huile, un gel anhydre, solide ou souple. De préférence, elle se présente sous forme anhydre, et plus spécialement sous forme de gel anhydre, notamment coulée en stick ou en coupelle.

Par "polymère semi-cristallin", on entend au sens de l'invention, des polymères comportant une partie cristallisable, chaîne pendante ou séquence dans le squelette, et une partie amorphe dans le squelette et présentant une température de changement de phase réversible du premier ordre, en particulier de fusion (transition solide-liquide). Par "polymères", on entend au sens de l'invention des composés comportant au moins 2 motifs de répétition, de préférence au moins 3 motifs de répétition et plus spécialement au moins 10 motifs répétitifs. Lorsque la partie cristallisable est une séquence du squelette polymérique, cette séquence cristallisable est de nature chimique différente de celle des séquences amorphes ; le polymère semi-cristallin est dans ce cas un polymère séquencé par exemple du type dibloc, tribloc ou multibloc

De façon avantageuse, la composition selon l'invention comprend, de plus, au moins un composé cristallin ou semi-cristallin à structure organique et à haut point de fusion, solide à température ambiante et ayant une température de fusion au moins égale à 50°C. Par "composé organique" ou "à structure organique", on entend des composés contenant des atomes de carbone et des atomes d'hydrogène et éventuellement des hétéroatomes comme S, O, N, P seuls ou en association.

Dans la suite de la description, le ou les polymères semi-cristallins ayant une température de fusion Pf₂ inférieure à 50°C seront dénommés "polymères à bas point de fusion" et le ou les composés cristallins ou semi-cristallins ayant une température de

Dans le document US-A-5 302 380 (DA), il est décrit des compositions cosmétiques d'adhérence améliorée sur la peau, contenant des homopolymères de polypropylène atactique de cristallinité de 0,1 à 15 % et de poids moléculaire d'environ 1 000 à 10 000. Ces polymères ne comportent ni chaîne pendante cristallisable ni séquence cristallisable. Leur agencement atactique, à savoir irrégulier, ne permet normalement pas une cristalisation. En outre, ces polymères n'assurent nullement la structuration des compositions les contenant.

Dans la suite de la description, le ou les polymères semi-cristallins ayant une température de fusion Pf₂ inférieure à 50°C seront dénommés "polymères à bas point de fusion" et le ou les composés cristallins ou semi-cristallins ayant une température de fusion Pf₁ supérieure ou égale à 50°C seront dénommés "composés à haut point de fusion". Selon l'invention, le point de fusion peut être mesuré notamment par toute méthode connue et en particulier avec un calorimètre à balayage différentiel (D.S.C).

La structuration ou gélification de la phase grasse liquide selon l'invention est due à une cristallisation du ou des polymères semi-cristallins dans la phase grasse liquide, associés ou non à un ou plusieurs composés cristallins et non pas à une interaction physique du type dipôle ou hydrogène, entre chaînes polymériques, telle que décrite dans les documents US-A-5 318 995 (D1) et EP-A-0 550 745 (D2). Pour obtenir un épaississement élevé selon D1 et D2, il est nécessaire de former un réseau réticulé, fortement interpénétré, par interactions physiques entre les chaînes des polymères. Ceci est obtenu en utilisant des polymères de masse moléculaire moyenne en nombre supérieure à 100 000. Cette forte réticulation physique des chaînes polymériques conduit à des gels cassants, rigides, qui ne permettent pas le dépôt d'un film sur les matières kératiniques et sont difficiles à prendre au doigt. De plus, certains des polymères décrits dans ces documents ne sont pas ou peu solubles dans les huiles classiquement utilisées en cosmétique, ce qui limite leur utilisation.

Comme composé à haut point de fusion utilisable dans l'invention, on peut citer les cires à haut point de fusion comme certaines cires de polyéthylène telle que l'Epolène N-14 vendu par Eastman Chemical Cie, les cires de Carnauba et certaines cires microcristallines comme celles vendues par Tisco sous la marque "Tisco wax 88", ainsi que des polymères semi-cristallins à haut point de fusion. Les polymères semi-cristallins à haut point de fusion sont en particulier des polymères semi-cristallins à structure organique, solides à température ambiante et ayant une température de fusion supérieure ou égale à 50°C, comportant a) un squelette organique polymérique et b) au moins une chaîne organique latérale cristallisable et/ou une séquence organique cristallisable faisant partie dudit squelette polymérique, ledit polymère ayant une masse moléculaire moyenne en nombre supérieure à 2 000.

De préférence, le composé à haut point de fusion est un second polymère semi-cristallin solide organique à haut point de fusion. Il est toutefois possible d'utiliser comme composé à haut point de fusion des polymères cristallins, solides à température ambiante et ayant une température de fusion supérieure à 50°C des polymères statistiques comportant une cristallisation contrôlée, tels que décrits dans le document (D3) EP-A-0 951 897 et plus spécialement les produits commerciaux Engage 8 401 et Engage 8 402 de Dupont de Nemours, respectivement de température de fusion de 51°C et 64°C et qui sont des bipolymères statistiques éthylène/1-octène.

L'association d'un ou plusieurs composés à haut point de fusion et notamment de polymères semi-cristallins à haut point de fusion à un ou plusieurs polymères à bas point de fusion permet de conférer à la composition une bonne stabilité dans le temps et en température. Ainsi, il est possible d'obtenir une composition restant macroscopiquement homogène, sans exsudation de la phase grasse liquide, même en atmosphère humide, pendant au moins 2 mois à 25°C et pression atmosphérique.

De plus, les propriétés de non-migration de la composition dans les rides et ridules de la peau notamment autour des lèvres, mais aussi dans les plis de la paupière supérieure et autour des yeux, sont améliorées.

### Les polymères semi-cristallins à haut et bas point de fusion

De façon avantageuse, le ou les polymères semi-cristallins (à haut ou bas point de fusion) de la composition de l'invention comprennent une masse moléculaire moyenne en nombre M̅n allant de 2 000 à 800 000, de préférence de 3 000 à 500 000, par exemple de 4 000 à 150 000 notamment inférieure à 100 000, et mieux de 4 000 à 99 000. De préférence, ils présentent une masse moléculaire moyenne en nombre supérieure à 5 600, allant par exemple de 5 700 à 99 000.

Le ou les polymères semi-cristallins selon l'invention servant d'agent structurant sont des solides, non déformables à température ambiante (25°C) et pression atmosphérique (760 mm de Hg). Ils sont capables de structurer, seuls ou en mélange, la composition sans ajout de tensioactif particulier, ni de charge, ni de cire.

Selon l'invention les polymères semi-cristallins à bas point de fusion et/ou les composés à haut point de fusion sont avantageusement solubles dans la phase grasse à au moins 1 % en poids, à une température supérieure à leur température de fusion. En dehors des chaînes ou séquences cristallisables, les séquences des polymères sont amorphes. Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère.

De préférence, le squelette polymérique des polymères semi-cristallins est soluble dans la phase grasse liquide.

Selon l'invention le ou les composés semi-cristallins à haut point de fusion sont avantageusement des polymères ayant une température de fusion Pf₁ telle que 50°C ≤ Pf₁ ≤ 150°C, mieux 55°C ≤ Pf₁ ≤ 150°C, et de préférence 60°C ≤ Pf₁ ≤ 130°C et les polymères à bas point de fusion ont avantageusement une température de fusion Pf₂ telle que 30°C ≤ Pf₂ < 50°C, et mieux 35°C ≤ Pf₂ ≤ 45°C. Cette température de fusion est une température de changement d'état du premier ordre.

De façon générale, les polymères à bas point de fusion présentent une température de fusion Pf₂ au moins égale à la température du support kératinique devant recevoir la composition selon l'invention.

De préférence, la ou les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristalisables, ces derniers sont avantageusement sous forme aléatoire ou statistique.

De préférence, les polymères semi-cristallins de l'invention sont d'origine synthétique. En outre, ils ne comportent pas de squelette polysaccharidique. De façon générale, les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins.

Selon l'invention, le polymère semi-cristallin à bas point de fusion et le polymère semi-cristallin à haut point de fusion sont choisis parmi les copolymères séquencés comportant au moins une séquence cristallisable et au moins une séquence amorphe, les homopolymères et les copolymères portant au moins une chaîne latérale cristallisable par motif de répétition, leurs mélanges.

Les polymères semi-cristallins utilisables dans l'invention sont en particulier :
- les copolymères séquencés de polyoléfines à cristallisation contrôlée, notamment ceux dont les monomères sont décrits dans D3.
- les polycondensats et notamment de type polyester, aliphatique ou aromatique ou copolyester aliphatique/aromatique,
- les homo- ou co-polymères portant au moins une chaîne latérale cristallisable et les homo- ou co-polymères portant dans le squelette au moins une séquence cristallisable, comme ceux décrits dans le document (D4) US-A-5 156 911,
- les homo- ou co-polymères portant au moins une chaîne latérale cristallisable en particulier à groupement(s) fluoré(s), tels que décrits dans le document (D5) WO-A-01/19333,
- et leurs mélanges. Dans ces deux derniers cas, la ou les chaînes latérales ou séquences cristallisables sont hydrophobes.

### A) Polymères semi-cristallins à chaînes latérales cristallisables

On peut citer en particulier ceux définis dans les documents D4 et D5.
- Ce sont des homopolymères ou copolymères comportant de 50 à 100 % en poids de motifs résultant de la polymérisation d'un ou plusieurs monomères porteurs de chaîne latérale hydrophobe cristallisable.
- Ces homo- ou co-polymères sont de toute nature du moment qu'ils présentent les conditions indiquées ci-après avec en particulier la caractéristique d'être solubles ou dispersables dans la phase grasse liquide, par chauffage au-dessus de leur température de fusion Pf. Ils peuvent résulter :
   - de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
   - de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

Ces polymères sont choisis notamment parmi les homopolymères et copolymères résultant de la polymérisation d'au moins un monomère à chaîne(s) cristallisable(s) qui peut être représenté par la formule X : 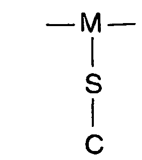 avec M représentant un atome du squelette polymérique
S représentant un espaceur
C représentant un groupe cristallisable

Les chaînes « -S-C » cristallisables peuvent être aliphatiques ou aromatiques, éventuellement fluorées ou perfluorées. « S » représente notamment un groupe (CH₂)ₙ ou (CH₂CH₂O)ₙ ou (CH₂O), linéaire ou ramifié ou cyclique, avec n entier allant de 0 à 22. De préférence « S » est un groupe linéaire. De préférence, « S » et « C » sont différents.

Lorsque les chaînes « -S-C » cristalisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyle en C₁₄-C₂₄. Lorsqu'il s'agit de chaînes alkyle fluorées ou perfluorées, elles comportent au moins 6 atomes de carbone fluorés et notamment au moins 11 atomes de carbone dont au moins 6 atomes de carbone sont fluorés.

Comme exemple de polymères ou copolymères semi-cristallins à chaîne(s) cristallisable(s), on peut citer ceux résultant de la polymérisation d'un ou plusieurs monomères suivants : les (méth)acrylates d'alkyle saturés avec le groupe alkyle en C₁₄-C₂₄, les (méth)acrylates de perfluoroalkyle avec un groupe alkyle perfluoro en C₁₁-C₁₅. les N-alkyl (méth)acrylamides avec le groupe alkyle en C₁₄ à C₂₄ avec ou sans atome de fluor, les esters vinyliques à chaînes alkyle ou perfluoro (alkyle) avec le groupe alkyle en C₁₄ à C₂₄ (avec au moins 6 atomes de fluor pour une chaîne perfluoro alkyle), les éthers vinyliques à chaînes alkyle ou perfluoro (alkyle) avec le groupe alkyle en C₁₄ à C₂₄ et au moins 6 atomes de fluor pour une chaîne perfluoro alkyle, les alpha-oléfines en C₁₄ à C₂₄ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50 % de groupes Y ou Z résultant de la copolymérisation :
α) de Y qui est un monomère polaire ou non polaire ou un mélange des deux :
   - Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un NN-dialkyl(méth)acrylamide comme par exemple le NN-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
   - Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle en C₁ à C₁₀, comme l'α-méthylstyrène, un macromonomère du type polyorganosiloxane à insaturation vinylique. Par "alkyle", on entend au sens au sens de l'invention un groupement saturé notamment en C₈ à C₂₄, sauf mention exprès, et mieux en C₁₄-C₂₄.
β) de Z qui est un monomère polaire ou un mélange de monomères polaires. Dans ce cas, Z a la même définition que le "Y polaire" défini ci-dessus.

De préférence, les polymères semi-cristallins à chaîne latérale cristallisable sont des homopolymères d'alkyl(méth)acrylate ou d'alkyl(méth)acrylamide avec un groupe alkyle tel que défini ci-dessus, et notamment en C₁₄-C₂₄, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

### B) Les polymères portant dans le squelette au moins une séquence cristallisable

Il s'agit encore de polymères solubles ou dispersables dans la phase grasse liquide par chauffage au-dessus de leur point de fusion Pf. Ces polymères sont notamment des copolymères séquencés constitués d'au moins 2 séquences de nature chimique différente dont l'une est cristallisable.
- On peut utiliser les polymères définis dans le brevet US-A-5 156 911 (D4),
- Les copolymères séquencés d'oléfine ou de cyclooléfine à chaîne cristallisable comme ceux issus de la polymérisation séquencée de :
   - cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
   - avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
   - et en particulier les copoly(éthylène/norbornène) blocs et les terpolymères (éthylène/propylène/éthylidène-norbornène), blocs. On peut aussi utiliser ceux résultants de la copolymérisation séquencée d'au moins 2 α-oléfines en C₂-C₁₆ et mieux en C₂-C₁₂ tels que ceux cités précédemment et en particulier les bipolymères séquencés d'éthylène et d'1-octène.
- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
   - Séquence cristallisable par nature : a) polyester comme les poly(alkylène téréphtalate), b) polyoléfine comme les polyéthylènes ou polypropylènes.
   - Séquence amorphe et lipophile comme : les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :
α) les copolymères séquencés poly(ε-caprolactone)-b-poly(butadiène), utilisés de préférence hydrogénés, tels que ceux décrits dans l'article D6 "Melting behavior of poly(-caprolactone)-block-polybutadiène copolymers" de S. Nojima, Macromolécules, 32, 3727-3734 (1999).
β) les copolymères séquencés poly(butylènetéréphtalate)-b-poly(isoprène) hydrogénés séquencés ou multiséquencés, cités dans l'article D7 "Study of morphological and mechanical properties of PP/PBT" de B. Boutevin et al., Polymer Bulletin, 34, 117-123 (1995).
γ) les copolymères séquencés poly(éthylène)-b-copoly(éthylène/propylène) cités dans les articles D8 "Morphology of semi-crystalline block copolymers of ethylene-(ethylene-alt-propylene)" de P. Rangarajan et al., Macromolecules, 26, 4640-4645 (1993) et D9 "Polymer agregates with crystalline cores : the system poly(ethylene)-poly(ethylene-propylene)" P. Richter et al., Macromolécules, 30, 1053-1068 (1997).
δ) les copolymères séquencés poly(éthylène)-b-poly(éthyléthylène) cités dans l'article général D10 "Cristallization in block copolymers" de I.W. Hamley, Advances in Polymer Science, vol 148, 113-137 (1999).

Les polymères semi-cristallins de la composition de l'invention peuvent être ou non réticulés en partie du moment que le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.

De préférence, les polymères semi-cristallins de la composition selon l'invention sont non réticulés.

Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en C₁₄-C₂₄, les (méth)acrylates de perfluoroalkyle en C₁₁-C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ éventuellement fluoré, qui peut être représenté par la formule suivante : 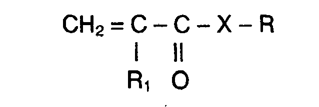 dans laquelle R₁ est H ou CH₃, R représente un groupe alkyle en C₁-C₁₀ éventuellement fluoré et X représente O, NH ou NR₂, où R₂ représente un groupe alkyle en C₁-C₁₀ éventuellement fluoré.

Selon un mode plus particulier de réalisation de l'invention, le polymère est issu d'un monomère à chaîne cristallisable choisi parmi les (méth)acrylates d'alkyle saturés en C₁₄-C₂₂.

A titre d'exemple particulier de polymère semi-cristallins structurant utilisable dans la composition selon l'invention, on peut citer les produits Intelimer® de la société Landec décrits dans la brochure D₁₁ "Intelimer® polymers", Landec IP22 (Rev. 4-97). Ces polymères sont sous forme solide à température ambiante (25°C). Ils sont porteurs de chaînes latérales cristallisables et présentent la formule X précédente.
i) Les polymères semi-cristallins à bas point de fusion sont notamment :
   ceux décrits dans les exemples 3, 4, 5, 7, 9 du brevet (D4) US-A-5 156 911 à groupement -COOH, résultant de la copolymérisation d'acide acrylique et d'alkyl(méth)acrylate en C₅ à C₁₆ de Pf₂ allant de 20°C à 35°C et plus particulièrement de la copolymérisation :
   - d'acide acrylique, d'hexadécylacrylate et d'isodécylacrylate dans un rapport 1/16/3,
   - d'acide acrylique et de pentadécylacrylate dans un rapport 1/19,
   - d'acide acrylique, d'hexadécylacrylate, éthylacrylate dans un rapport 2,5/76,5/20,
   - d'acide acrylique, d'hexadécylacrylate et de méthylacrylate dans un rapport 5/85/10, .
   - d'acide acrylique, de polyoctadécylméthacrylate dans un rapport 2,5/97,5.

   On peut aussi utiliser le polymère Structure « O » de National Starch tel que celui décrit dans le document D12 (US-A-5 736 125) de Pf₂ de 44°C ainsi que les polymères semi-cristallins à chaînes pendantes cristalisables comportant des groupements fluorés tels que décrits dans les exemples 1, 4, 6, 7 et 8 du document (D5).
   On peut encore utiliser les polymères semi-cristallins de bas point de fusion obtenus par copolymérisation d'acrylate de stéaryle et d'acide acrylique ou de NVP tels que décrits dans le document D13 (US-A-5 519 063) ou D2 (EP-A- 055 0745) et plus spécialement ceux décrits dans les exemples 1 et 2, ci-après, de préparation de polymère, de température de fusion respectivement de 40°C et 38°C.
ii) Les polymères semi-cristallins à haut point de fusion sont notamment l'Intelimer décrit dans le document D11 de température de fusion Pf₁ de 56°C, qui est un produit visqueux à température ambiante, imperméable, non-collant.

On peut aussi utiliser les polymères semi-cristallins obtenus par copolymérisation de l'acrylate de béhényle et de l'acide acrylique ou de NVP, tels que décrits dans les documents D13 et D2 et plus spécialement ceux décrits dans les exemples 3 et 4, ci-après, de préparation de polymère, de température de fusion respectivement de 60°C et 58°C.

De préférence, les polymères semi-cristallins à bas point de fusion et/ou ceux à haut point de fusion ne comportent pas de groupement carboxylique.

La gélification de la phase grasse est modulable selon la nature des polymères et leurs concentrations respectives et peuvent être telles que l'on obtienne une structure rigide sous forme d'un bâton ou d'un stick.

Le taux de chaque polymère est choisi selon la dureté de la composition désirée et en fonction de l'application particulière envisagée. Les quantités respectives de polymère peuvent être telles qu'elles permettent l'obtention d'un solide délitable, présentant en particulier une dureté allant de 100 à 350 gf. Cette dureté peut être mesurée par la méthode dite du "fil à couper le beurre", qui consiste à couper un bâton de rouge à lèvres de 12,7 mm de diamètre et à mesurer la dureté à 20°C, au moyen d'un dynamomètre DFGHS 2 de la société Indelco-Chatillon se déplaçant à une vitesse de 100mm/minute. Elle est exprimée comme la force de cisaillement (exprimée en gramme-force) nécessaire pour couper un stick dans ces conditions.

Cette dureté est telle que la composition est autoportée et peut se déliter aisément pour former un dépôt satisfaisant sur la peau et les lèvres. En outre, avec cette dureté, la composition de l'invention sous forme coulée notamment en stick résiste bien aux chocs.

De préférence, la composition de l'invention se présente sous forme d'un stick solide, de dureté allant de 100 gf à 350 gf, mesurée selon la méthode du "fil à couper le beurre". Il est toutefois possible d'utiliser une quantité de polymère semi-cristallin telle que la composition soit sous forme de pâte souple applicable au doigt ou à l'aide d'un applicateur sur les matières kératiniques.

En pratique, la quantité totale de polymère semi-cristallin représente de 0,1 à 80 % du poids total de la composition et mieux de 0,5 à 40 % et encore mieux de 3 à 30 %. De préférence, il représente plus de 10 % en poids de la composition.

Selon l'invention et de façon avantageuse, le composé à haut point de fusion (cristallin ou semi-cristallin) et celui à bas point de fusion sont dans un rapport en poids allant de 10/90 à 90/10 et mieux de 40/60 à 60/40.

De façon avantageuse, le rapport pondéral de polymère semi-cristallin à structure organique par rapport à la phase grasse liquide est de 0,20 à 0,60 et mieux de 0,25 à 0,50, pour obtenir un stick dur qui se délite au contact de la peau ou des lèvres et en particulier de dureté allant de 100 à 350 gf.

Les bâtons ou sticks selon l'invention, lorsqu'ils sont colorés permettent, après application, d'obtenir un dépôt brillant, homogène en couleur, non collant, de bonne couvrance (c'est-à-dire que la peau ou les lèvres n'apparaît pas sous le maquillage).

### La phase grasse liquide

Avantageusement, la phase grasse liquide, structurée par les polymères semi-cristallins à bas point de fusion et/ou les polymères semi-cristallins à haut point de fusion, constitue la phase continue de la composition. Cette phase grasse peut contenir une ou plusieurs huiles apolaires ou non ou un mélange d'huile(s) apolaire(s) et d'huile(s) polaire(s).

Les huiles apolaires selon l'invention sont en particulier les huiles siliconées telles que les polydiméthylsiloxanes (PDMS), linéaires ou cycliques, liquides à température ambiante ; les polydiméthylsiloxanes comportant des groupements alkyle, alcoxy ou phényle, pendant et/ou en bout de chaîne siliconée, groupements ayant de 2 à 24 atomes de carbone, liquides à température ambiante ; les silicones phénylées comme les phényl triméthicones, les phényl diméthicones, les phényl triméthylsiloxy diphénylsiloxanes, des diphényl diméthicones, les diphényl méthyldiphényl trisiloxanes, les 2-phényléthyl triméthylsiloxysilicates, liquides ; les hydrocarbures ou fluorocarbures linéaires ou ramifiés d'origine synthétique ou minérale, liquides, comme les huiles de paraffine et leurs dérivés, la vaseline, les polydécènes, le polyisobutène hydrogéné tel que le Parléam® vendu par la société Nippon Oil Fats, le squalane ; leurs mélanges. De préférence, les huiles apolaires utilisées sont des huiles apolaires du type hydrocarboné, liquides, d'origine minérale ou synthétique, choisies notamment parmi l'huile de Parleam® (isoparaffine hydrogénée), les isoparaffines, le squalane et leurs mélanges. Avantageusement, la phase grasse liquide contient au moins une huile hydrocarbonée d'origine minérale ou synthétique.

Par "huile hydrocarbonée", on entend au sens de l'invention des huiles contenant majoritairement des atomes de carbone et des atomes d'hydrogène et en particulier des chaînes alkyle ou alcényle comme les alcanes ou alcènes mais aussi les huiles à chaîne alkyle ou alcényle comportant un ou des groupements éther, ester, hydroxyle ou acide carboxylique.

Il est possible d'ajouter aux huiles apolaires des huiles polaires, les huiles apolaires servant notamment de cosolvant des huiles polaires.

En particulier, les huiles polaires de l'invention sont :
- les huiles végétales hydrocarbonées à forte teneur en triglycérides constitués d'esters d'acides gras (en C₈ à C₂₄) et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les huiles de synthèse de formule R₅COOR₆ dans laquelle R₅ représente le reste d'un acide gras supérieur linéaire ou ramifié comportant de 7 à 40 atomes de carbone et R₆ représente une chaîne hydrocarbonée ramifiée contenant de 3 à 40 atomes de carbone comme par exemple l'huile de Purcellin (octanoate de cétostéaryle), l'isononanoate d'isononyle, le benzoate d'alcool en C₁₂ à C₁₅ ;
- les esters et les éthers de synthèse comme le myristate d'isopropyle, le palmitate d'éthyl 2-hexyle, des octanoates, décanoates ou ricinoléates d'alcools ou de polyalcools, les esters hydroxylés comme le lactate d'isostéaryle, le malate de di-isostéaryle ; et les esters du pentaérythritol ;
- les alcools gras en C₈ à C₂₆ comme l'alcool oléique ;
- les acides gras ayant de 12 à 22 atomes de carbone comme l'acide oléique, linoléique
ou linolénique ;
- leurs mélanges.
   La phase grasse représente, en pratique, de 5 à 99 % du poids total de la composition, de préférence de 20 à 80 %. Avantageusement, elle représente au moins 60 % du poids total de la composition.

### Les additifs

La composition de l'invention peut comprendre, en outre, tout additif usuellement utilisé dans le domaine concerné, choisi parmi l'eau éventuellement épaissie par un épaississant ou gélifiant de phase aqueuse, les matières colorantes, les antioxydants, les huiles essentielles, les conservateurs, les parfums, les charges, les dispersants, les corps gras pâteux ou les cires autres que les composés à haut point de fusion, les neutralisants, leurs mélanges. Ces additifs peuvent être présents dans la composition selon les quantités généralement utilisées dans le domaine cosmétique et dermatologique et notamment à raison de 0,01 à 50% du poids total de la composition et mieux de 0,1 à 20%. L'eau peut représenter jusqu'à 70 % du poids total de la composition.

Bien entendu l'homme du métier veillera à choisir les éventuels additifs complémentaires et/ou leur quantité de telle manière que les propriétés avantageuses de la composition selon l'invention, à savoir brillance, non collant, couvrance et non-migration notamment ne soient pas ou substantiellement pas, altérées par l'adjonction envisagée.

La composition selon l'invention peut se présenter sous la forme d'une composition dermatologique ou de soin de la peau et/ou des phanères ou sous forme d'une composition de protection solaire, de soin du visage ou du corps, d'hygiène corporelle, notamment sous forme de déodorant. Elle se présente alors notamment sous forme non colorée. Elle peut alors être utilisées comme base de soin pour la peau, les phanères ou les lèvres (baumes à lèvres, protégeant les lèvres du froid et/ou du soleil et/ou du vent, crème de soin pour la peau, les ongles ou les cheveux), d'un shampoing ou d'un après shampoing, d'un produit de protection solaire.

La composition de l'invention peut également se présenter sous la forme d'un produit coloré en particulier de maquillage de la peau, présentant éventuellement des propriétés de soin ou de traitement, et être en particulier un fond de teint, un blush, un fard à joues ou à paupières, un produit anti-cerne, un eye-liner, un produit de maquillage du corps ; de maquillage des lèvres comme un rouge à lèvres, un brillant à lèvres ou un crayon à lèvres présentant éventuellement des propriétés de soin ou de traitement ; de maquillage des phanères comme les ongles, les cils sous forme de mascara, les sourcils et les cheveux.

Bien entendu la composition de l'invention doit être cosmétiquement ou dermatologiquement acceptable, à savoir contenir un milieu physiologiquement acceptable non toxique et susceptible d'être appliqué sur la peau, les phanères ou les lèvres du visage d'êtres humains. Par cosmétiquement acceptable, on entend au sens de l'invention une composition d'aspect, d'odeur, de toucher et éventuellement de goût agréables.

Avantageusement, la composition contient une matière colorante qui peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 50 % (en matière sèche) du poids total de la composition, de préférence de 5 à 30 % (si présente). Par pigments et nacres, on entend des particules solides et insolubles à température ambiante dans le milieu physiologiquement acceptable de la composition.

Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,01 à 6 % (si présents). Les colorants hydrosolubles sont par exemple le jus de betterave, le bleu de méthylène et peuvent représenter jusqu'à 6 % du poids total de la composition.

Les pigments peuvent être blancs ou colorés, minéraux et/ou organiques, sphériques ou non, plaquétaires ou non, enrobés ou non. On peut citer, parmi les pigments minéraux, le dioxyde de titane ou de zinc, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium (tels que le D & C red 27, 21, 7, D & C yellow 5, 6, le F D & C blue n°1). Les pigments peuvent représenter de 0 à 40 % (0,01 à 40 %) notamment de 0,5 à 35 % et mieux de 2 à 25 % du poids total de la composition (si présents).

Les pigments nacrés (ou nacres) peuvent être choisis parmi les pigments nacrés blancs tels que le mica notamment recouvert de titane ou d'oxychlorure de bismuth, les pigments nacrés colorés tels que le mica-titane avec des oxydes de fer, le mica-titane avec notamment du bleu ferrique ou de l'oxyde de chrome, le mica-titane avec un pigment organique du type précité ainsi que les pigments nacrés à base d'oxychlorure de bismuth ou les pigments interférentiels, notamment à cristaux liquides ou multicouches. Ils peuvent représenter de 0 à 25 % (0,05 à 25 %) du poids total de la composition et mieux de 0,1 à 15 % (si présents).

De préférence, la matière colorante comprend des pigments et/ou des nacres.

Avantageusement, les pigments et les nacres sont introduits dans la composition sous forme de pâte pigmentaire.

Par "pâte pigmentaire", on entend au sens de l'invention une dispersion colloïdale concentrée de particules solides à température ambiante colorées enrobées ou non dans un milieu continu, stabilisée en surface à l'aide d'un agent dispersant ou éventuellement sans agent dispersant.

L'agent dispersant sert à protéger les particules dispersées contre leur agglomération ou floculation. La concentration en dispersant généralement utilisée pour stabiliser des particules solides en dispersion et notamment une dispersion colloïdale est de 0,3 à 5 mg/m², de préférence de 0,5 à 4 mg/m², de surface de particules. Cet agent dispersant peut être un tensioactif, un oligomère, un polymère ou un mélange de plusieurs d'entre eux, portant une ou des fonctionnalités ayant une affinité forte pour la surface des particules à disperser. Il peut s'accrocher physiquement ou chimiquemenet à la surface des particules à disperser. En outre, il présente au moins un groupe fonctionnel compatible ou soluble dans le milieu continu. En particulier, on utilise les esters de l'acide poly(12)hydroxystéarique comme le stéarate d'acide poly(12-hydroxystéarique) tel que celui vendu sous le nom de Solsperse 21 000 par la société Avecia, le polyglyceryl-2 dipolyhydroxystearate (nom CTFA) vendu sous la référence Dehymuls PGPH par la société HENKEL, l'acide poly(12-hydroxystéarique) vendu sous la référence Arlacel P100 par la société Uniqema, et leurs mélanges. Ces agents dispersants peuvent, en outre, être utilisé comme additif, sans pour autant faire partie d'une pâte particulaire ou pigmentaire.

La dispersion colloïdale est une suspension de particules de taille généralement micronique (<10 µm) dans un milieu continu. La fraction volumique de particules dans une dispersion concentrée est de 20 % à 40 %, de préférence supérieure à 30 %.

Les particules dispersées dans le milieu peuvent être constituées de particules minérales ou organiques ou de leurs mélanges tels que ceux décrits précédemment.

Le milieu continu de la pâte pigmentaire peut être quelconque et contenir tout solvant ou corps gras liquide et leurs mélanges. Avantageusement, le milieu liquide de la pâte pigmentaire est l'une des huiles que l'on souhaite utiliser dans la composition.

On peut aussi utiliser dans la composition de l'invention au moins une cire telle que celles utilisées jusqu'à ce jour en cosmétique.

Une cire, au sens de la présente invention, est un composé gras lipophile, solide à température ambiante (25°C) et pression atmosphérique (760 mm de Hg), à changement d'état solide/liquide réversible, ayant une température de fusion supérieure à 40°C et mieux supérieure à 50°C, pouvant aller jusqu'à 200° C, et présentant à l'état solide une organisation cristalline anisotrope. La taille des cristaux est telle que les cristaux diffractent et/ou diffusent la lumière, conférant à la composition un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange. Cette recristallisation dans le mélange peut être responsable de la diminution de la brillance dudit mélange. Aussi, avantageusement, la composition contient peu ou pas de cires classiques et notamment moins de 10 %, en poids, de cire classique et mieux moins de 5% par rapport au poids total de la composition.

Les cires classiques, au sens de la demande, sont celles généralement utilisées dans les domaines cosmétique et dermatologique ; elles sont notamment d'origine naturelle comme la cire d'abeilles, de Candellila, d'Ouricoury, du Japon, de fibres de liège ou de canne à sucre, les cires de paraffine, de lignite, les cires microcristallines de point de fusion > 50°C, la cire de lanoline, la cire de Montan, les ozokérites, les huiles hydrogénées comme l'huile de jojoba hydrogénée, mais aussi d'origine synthétique comme les cires de polyéthylène issues de la polymérisation de l'éthylène et les cires obtenues par synthèse de Fischer-Tropsch à point de fusion > 50°C, les esters d'acides gras et les glycérides concrets à 50°C, les cires de silicone comme les alkyle, alcoxy et/ou esters de poly(di)méthylsiloxane solide à 50°C.

Avantageusement, la composition de l'invention contient peu ou pas de charges "matifiantes" et en particulier moins de 5 % de charge matifiante. Ceci est notamment le cas lorsqu'on souhaite obtenir un dépôt brillant sur les matières kératiniques comme les lèvres, les cils et les cheveux. Pour un fond de teint, on peut au contraire utiliser ce type de charges. Une charge matifiante est en général une charge qui absorbe la sueur et/ou le sébum de la peau comme les silices, talcs, argiles, kaolins, poudres de polyamide (Nylon®), l'amidon.

La composition selon l'invention peut être fabriquée par les procédés connus, généralement utilisés dans le domaine cosmétique ou dermatologique. Elle peut être fabriquée par le procédé qui consiste à chauffer le polymère au moins à sa température de fusion, à y ajouter le ou les éventuels composés amphiphiles, les matières colorantes solubles dans le milieu, les pâtes pigmentaires et les additifs puis à mélanger le tout jusqu'à l'obtention d'une solution claire, translucide. Le mélange homogène obtenu peut alors être coulé dans un moule approprié comme un moule de rouge à lèvres ou directement dans les articles de conditionnement (boîtier ou coupelle notamment).

Avantageusement, la composition de l'invention est un rouge à lèvres contenant au moins une phase grasse liquide structurée par au moins un polymère à bas point de fusion semi-cristallin à structure organique, solide à température ambiante et ayant une température de fusion inférieure à 50°C, comportant a) un squelette polymérique et b) au moins une chaîne organique latérale cristallisable et/ou une séquence organique cristallisable faisant partie du squelette polymérique du polymère à bas point de fusion, ledit polymère ayant une masse moléculaire moyenne en nombre supérieure à 2 000, la phase grasse liquide et le polymère formant un milieu physiologiquement acceptable pour les lèvres. Avantageusement, ce rouge à lèvres contient une phase grasse continue constituée de tout ou partie de la phase grasse liquide structurée. Avantageusement, ce rouge à lèvres contient un composé de haut point de fusion tel que décrit précédemment et en particulier un second polymère semi-cristallin.

L'invention a encore pour objet un procédé cosmétique de soin, de maquillage ou de traitement des matières kératiniques des êtres humains et notamment de la peau, des lèvres du visage et des phanères des êtres humains, comprenant l'application sur les matières kératiniques de la composition notamment cosmétique telle que définie ci-dessus.

L'invention a encore pour objet l'utilisation cosmétique d'une quantité suffisante d'au moins un polymère à bas point de fusion semi-cristallin à structure organique, solide à température ambiante et ayant une température de fusion inférieure à 50°C, comportant a) un squelette polymérique et b) au moins une chaîne organique latérale cristallisable et/ou une séquence organique cristallisable faisant partie du squelette polymérique de polymère à bas point de fusion, ledit polymère ayant une masse moléculaire moyenne en nombre supérieure à 2 000, dans une composition notamment cosmétique contenant un milieu physiologiquement acceptable contenant au moins une phase grasse liquide continue, comme agent pour structurer, en l'absence de cire et/ou charge, la phase grasse liquide continue sous forme d'un solide de dureté allant de 100 à 350 gf. Il est possible, selon l'invention d'associer à ce polymère de bas point de fusion un composé cristallin ou semi-cristallin à structure organique et solide à température ambiante, ayant une température de fusion au moins égale à 50°C comme par exemple ceux cités précédemment.

L'invention a encore pour objet l'utilisation notamment cosmétique d'une quantité suffisante d'au moins un polymère semi-cristallin à structure organique et à haut point de fusion, solide à température ambiante et ayant une température de fusion au moins égale à 50°C, comportant i) un squelette polymérique et ii) au moins une chaîne organique latérale cristallisable et/ou séquence organique cristallisable faisant partie du squelette du polymère à haut point de fusion, ledit polymère ayant une masse moléculaire moyenne en nombre supérieure à 2 000, dans une composition notamment cosmétique contenant une phase grasse liquide continue, comme agent pour structurer, en l'absence de cire et/ou de charge, la phase grasse liquide continue sous forme d'un solide de dureté allant de 100 à 350 gf. Il est possible, selon l'invention d'associer à ce polymère de haut point de fusion un composé cristallin ou semi-cristallin à structure organique et solide à température ambiante ayant une température de fusion inférieure à 50°C comme par exemple ceux cités précédemment.

L'invention a encore pour objet l'utilisation cosmétique d'une quantité suffisante d'au moins un polymère semi-cristallin à structure organique à bas point de fusion, solide à température ambiante et ayant une température de fusion inférieure à 50°C, comportant a) un squelette polymérique et b) au moins une chaîne organique latérale cristallisable et/ ou une séquence organique cristallisable faisant partie du polymère à bas point de fusion, ledit polymère ayant une masse moléculaire moyenne en nombre supérieure à 2 000, dans une composition notamment cosmétique contenant un milieu physiologiquement acceptable et contenant une phase grasse liquide, pour structurer la phase grasse liquide sous forme d'un solide brillant et/ou non collant et/ou couvrant.

L'invention a encore pour objet l'utilisation notamment cosmétique de l'association d'un premier polymère semi-cristallin à bas point de fusion à structure organique solide à température ambiante et ayant une température de fusion inférieure à 50°C, comportant a) un squelette polymérique et b) au moins une chaîne organique latérale cristallisable et/ ou une séquence organique cristallisable faisant partie du premier polymère, ledit premier polymère ayant une masse moléculaire moyenne en nombre supérieure à 2 000, avec un second polymère semi-cristallin à structure organique et à haut point de fusion, solide à température ambiante et ayant une température de fusion au moins égale à 50°C, comportant i) un squelette polymérique et ii) au moins une chaîne organique latérale cristallisable et/ou une séquence organique cristallisable faisant patie du squelette du second polymère, ledit second polymère ayant une masse moléculaire moyenne en nombre supérieure à 2 000, dans une composition notamment cosmétique contenant un milieu physiologiquement acceptable, brillante et/ou non migrante et/ou non collante et/ou couvrante. Avantageusement, la composition est sous forme d'un solide brillant et/ou non collant et/ou couvrant. Il est possible de remplacer tout ou partie de ce second polymère par tout autre composé à haut point de fusion tel que décrit précédemment.

Avantageusement, la composition cosmétique selon l'invention présente des propriétés traitantes. En particulier l'association du polymère à bas point de fusion et du polymère à haut point de fusion peut être utilisée pour la fabrication d'une composition physiologiquement acceptable et en particulier dermatologique non migrante. Ainsi, il est possible de maintenir en place la composition là où elle a été déposée et d'améliorer ainsi son action locale et son efficacité.

L'invention est illustrée plus en détail dans les exemples suivants. Les quantités sont données en pourcentage massique.

### I) Exemples de fabrication de polymères semi-cristallins

### Exemple 1 : Polymère acide de point de fusion de 40°C

Dans un réacteur d'1l muni d'une agitation centrale avec ancre, d'un réfrigérant et d'un thermomètre, on introduit 120g de Parléam que l'on chauffe de la température ambiante à 80°C en 45 min. A 80°C, on introduit en 2h le mélange C₁ suivant :
40g de cyclohexane + 4g de Triganox 141 [ 2,5 bis (2-éthyl hexanoyl peroxy) - 2,5 - diméthyl hexane].
30 min après le début de la coulée du mélange C₁, on introduit en 1 h30 le mélange C₂ constitué de :
190g d'acrylate de stéaryle + 10g d'acide acrylique + 400 g de cyclohexane.
A la fin des deux coulées, on laisse agir 3h supplémentaires à 80°C puis on distille à pression atmosphérique la totalité du cyclohexane présent dans le milieu réactionnel.
On obtient alors le polymère à 60 % en poids en matière active dans le Parléam.
Sa masse moléculaire moyenne en poids M_{w} est de 35 000 exprimée en équivalent polystyrène et sa température de fusion T_{f} est de 40°C ± 1°C, mesurée par D.S.C.

### Exemple 2 : Polymère basique de point de fusion de 38°C

On applique le même mode opératoire que dans l'exemple 1, sauf que l'on utilise de la N-Vinyl Pyrrolidone en lieu et place de l'acide acrylique.
Le polymère obtenu est à 60% en poids en matière active dans le Parléam, sa masse moléculaire moyenne en poids M_{w} est de 38 000 et sa T_{f} de 38°C.

### Exemple 3 : Polymère Acide de point de fusion de 60°C

On applique le même mode opératoire que dans l'exemple 1, sauf que l'on utilise de l'acrylate de béhényle en lieu et place de l'acrylate de stéaryle. Le polymère obtenu est à 60% en poids en matière active dans le Parléam. Sa masse moléculaire moyenne en poids M_{w} est de 42 000 et sa T_{f} de 60°C.

### Exemple 4 : Polymère basique de point de fusion de 58°C

On applique le même mode opératoire que dans l'exemple 2, sauf que l'on utilise de l'acrylate de béhényle en lieu et place de l'acrylate de stéaryle. Le polymère obtenu est à 60% en poids en matière active dans le Parléam®. Son M_{w} est de 45 000 et sa T_{f} est de 58°C.

### II) Exemples de composition

### Exemple 5 : Formule de rouge à lèvres

- Copolymère acrylate de stéaryle/NVP 95/5 à 60% en matière active dans le Parléam selon exemple 2 10,1 %
- Copolymère acrylate de Béhényle/acide acrylique à 60% en matière active dans le Parléam selon exemple 3 10,1 %
- Pâte pigmentaire 17,7 %
- Isoparaffine hydrogénée (Parléam) qsp 100 %

*Préparation* : On solubilise (ou dissout) les polymères dans une partie de l'huile, à 100°C, puis on ajoute les pigments broyés au préalable à l'aide d'un broyeur tricylindre avec une partie de la phase huileuse. L'ensemble est mélangé à l'aide d'un barreau magnétique puis coulé dans des moules de rouge à lèvres. La pâte pigmentaire contient 49 % de pigments (D & C Red n°7 + Yellow n°6 (laque d'aluminium) + dioxyde de titane), 7,5 % de stéarate d'acide poly(12-hydroxystéarique) et 43,5 % de Parléam qui est une isoparaffine hydrogénée (6-8 moles d'isobutylène).

La pâte pigmentaire est obtenue à l'aide d'un disperseur-broyeur de type Dispermat et chauffage du parléam à 25 - 30°C pendant une trentaine de minutes. Elle est stable au moins 3 mois à température ambiante, c'est-à-dire qu'aucune sédimentation n'est observable même sous agitation.

On obtient un stick de rouge à lèvres ayant une dureté de 117 gf mesurée par la méthode du "fil à couper le beurre". Le rouge à lèvres obtenu est brillant, non collant et non migrant. Ceci a été confirmé par un test sensoriel en comparaison, par demi-lèvres avec un produit brillant de l'art antérieur Rouge Absolu de Lancôme. Le rouge à lèvres de l'invention a été jugé aussi brillant à l'application que celui de l'art antérieur pour l'ensemble des testeurs, avec une migration beaucoup plus faible.

### Exemple 6 : Formule de rouge à lèvres

- Copolymère selon l'exemple 3 12,5 %
- Copolymère selon l'exemple 1 12,5 %
- Pâte pigmentaire 17,7 %
- Isoparaffine hydrogénée qsp 100 %

La composition de la pâte pigmentaire est identique à celle de l'exemple 5.

Ce rouge à lèvres sous forme de stick a été réalisé comme dans l'exemple 5. Il est brillant, non collant et non migrant. Il a été jugé par un panel d'experts, en comparaison avec un rouge à lèvres de l'art antérieur Rouge Magnétique de Lancôme considéré comme peu migrant. Le rouge à lèvres de l'invention a été jugé plus brillant que Rouge Magnétique pour des propriétés de non-migration comparables.

Le rouge à lèvres de l'art antérieur Rouge Absolu et Rouge Magnétique ne contiennent pas de polymères semi-cristallins à bas de point de fusion, associés notamment à un composé cristallin ou semi-cristallin de haut point de fusion.

### Exemple 7 : Formule de rouge à lèvres

Il se différencie de l'exemple 5 par l'emploi d'une cire de polyéthylène (Performalen 500 vendu par la société Petrolite) de point de fusion de 83°C à 1°C près, à la place du polymère de l'exemple 3. Les propriétés cosmétiques obtenues sont comparables à celles de la formule de l'exemple 5.

### Exemple 8 : Formule de rouge à lèvres

- Engage 8400 10,0 %
- Copolymère de l'exemple 1 10,1 %
- Pâte pigmentaire 17,7 %
- Paraffine liquide hydrogénée qsp 100 %
   La pâte pigmentaire est identique à celle l'exemple 5.
   La fabrication de ce rouge à lèvres en stick est identique à celle de l'exemple 5.

### Exemple 9 : Formule de rouge à lèvres

Copolymère acrylate de stéaryle/acide acrylique (95/5) à 50% en matière active dans le Parléam 25 %

Copolymère acrylate de béhényle/N-vinyl pyrrolidone (95/5) à 62,5% en matière active dans le Parléam 25 %
- Solsperse 21000 (acide poly(12-hydroxystéarique) 2 %
- Pigments 8,66 %
- Isoparaffine hydrogénée qsp 100 %

*Préparation :* on solubilise (ou dissout) les polymères dans une partie de l'huile, à 100°C, puis on ajoute les pigments qui ont été broyés au préalable à l'aide d'un mélangeur tri cylindre avec une partie de la phase huileuse. L'ensemble est mélangé à l'aide d'un barreau magnétique puis coulé dans des moules de rouge à lèvres. Les pigments sont conformes à l'exemple 5.

On obtient un stick de rouge à lèvres ayant une dureté de 227gf mesurée par la méthode du "fil à couper le beurre", non migrant, non collant, se déposant facilement sur les lèvres et donnant un dépôt satiné.

### Exemple 10 : Formule de rouge à lèvres

Copolymère méthacrylate de béhényle/acide acrylique (95/5) à 50% dans le Parléam 25 %

Copolymère acrylate de béhényle/N-vinyl pyrrolidone (95/5) à 62,5% dans le Parléam 25 %
- Solsperse 21000 (acide poly(12-hydroxystéarique) 2 %
- Pigments 8,66 %
- Isoparaffine hydrogénée qsp 100 %

*Préparation* : on solubilise (ou dissout) les polymères dans une partie de l'huile, à 100°C, puis on ajoute les pigments qui ont été broyés au préalable à l'aide d'un mélangeur tri cylindre avec une partie de la phase huileuse. L'ensemble est mélangé à l'aide d'un barreau magnétique puis coulé dans des moules de rouge à lèvres. Les pigments sont conformes à l'exemple 5.

On obtient un stick de rouge à lèvres ayant une dureté de 342gf mesurée par la méthode du "fil à couper le beurre", non migrant, non collant, se déposant facilement sur les lèvres et donnant un dépôt satiné.

## Revendications

1. Composition contenant au moins une phase grasse liquide structurée par au moins un polymère à bas point de fusion semi-cristallin à structure organique solide à température ambiante et ayant une température de fusion inférieure à 50°C, comportant a) un squelette polymérique et b) au moins une chaîne organique latérale cristallisable et/ou une séquence organique cristallisable faisant partie du squelette polymérique dudit polymère, ledit polymère ayant une masse moléculaire moyenne en nombre supérieure à 2 000, la phase grasse liquide et le polymère formant un milieu physiologiquement acceptable,
**caractérisée en ce qu'**elle contient, en outre, un composé cristallin ou semi-cristallin à structure organique et à haut point de fusion, solide à température ambiante et ayant une température de fusion au moins égale à 50°C, et **en ce que** le polymère à bas point de fusion et/ou celui à haut point de fusion sont choisis parmi les homopolymères et copolymères résultant de la polymérisation d'au moins un monomère à chaîne(s) cristallisable(s) de formule X : 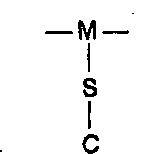 avec M représentant un atome du squelette polymérique
S représentant un espaceur
C représentant un groupe cristallisable
et leurs mélanges, avec « S-C » représentant une chaîne alkyle à au moins 11 atomes de carbone, éventuellement fluorée ou perfluorée.

2. Composition selon la revendication 1, **caractérisée en ce que** le composé cristallin ou semi-cristallin est un second polymère à haut point de fusion comportant i) un squelette polymérique et ii) au moins une chaîne latérale cristallisable et/ou une séquence organique cristallisable faisant partie du squelette dudit second polymère, ledit second polymère ayant une masse moléculaire moyenne en nombre supérieure à 2 000.

3. Composition selon la revendication 2, **caractérisée en ce** le polymère à bas point de fusion et/ou celui à haut point de fusion ont une masse moléculaire moyenne en nombre allant de 3 000 à 500 000 et mieux de 4 000 à 99 000.

4. Composition selon l'une quelconque des revendications 1 à 3 **caractérisée en ce que** le polymère à bas point de fusion et/ou le composé à haut point de fusion sont solubles dans la phase grasse liquide à une température supérieure à leur température de fusion.

5. Composition selon l'une des revendications précédentes, **caractérisée en ce que** le polymère à bas point de fusion a une température de fusion Pf₂ telle que 30°C ≤ Pf₂ < 50°C.

6. Composition selon l'une des revendications 1 à 5 **caractérisée en ce que** le composé à haut point de fusion a une température de.fusion Pf₁ telle que 55°C ≤ Pf₁≤ 150°C et de préférence 60°C ≤ Pf₁ ≤ 130°C.

7. Composition selon l'une des revendications 2 à 6 **caractérisée en ce que** le polymère à bas point de fusion et/ou celui à haut point de fusion sont choisis parmi les copolymères séquencés comportant au moins une séquence cristallisable et au moins une séquence amorphe, les homopolymères et les copolymères portant au moins une chaîne latérale cristallisable par motif de répétition, leurs mélanges.

8. Composition selon la revendication 7, **caractérisée en ce que** la séquence cristallisable est de nature différente de la séquence amorphe.

9. Composition selon l'une des revendications 2 à 7, **caractérisée en ce que** le polymère à bas point de fusion et/ou celui à haut point de fusion sont choisis parmi :
- les copolymères séquencés de polyoléfines à cristallisation contrôlée,
- les polycondensats polyesters aliphatiques ou aromatiques et les copolyesters aliphatiques/aromatiques,
- les homo- ou co-polymères portant au moins une chaîne latérale cristallisable,
- leurs mélanges.

10. Composition selon l'une des revendications 2 à 9 **caractérisée en ce** le polymère à bas point de fusion et/ou celui à haut point de fusion sont choisis parmi les homopolymères et copolymères comportant de 50 à 100% en poids de motifs résultant de la polymérisation de un ou plusieurs monomères porteurs de chaine(s) latérale(s) hydrophobe(s) cristallisable(s).

11. Composition selon l'une des revendications 2 à 10, **caractérisée en ce que** le polymère à bas point de fusion et/ou celui à haut point de fusion sont choisis parmi les polymères résultant de la polymérisation d'au moins un monomère choisis parmi l'acide acrylique, méthacrylique, crotonique, itaconique, maléique, l'anhydride maléique et leurs mélanges.

12. Composition selon l'une des revendications 2 à 11 **caractérisée en ce que** le polymère à bas point de fusion et/ou celui à haut point de fusion sont choisis parmi les homopolymères et copolymères résultant de la polymérisation d'au moins un monomère à chaîne cristallisable choisi parmi les (méth)acrylates d'alkyle saturés en C₁₄-C₂₄, les (méth)acrylates de perfluoroalkyle en C₁₁-C₁₆, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, leurs mélanges.

13. Composition selon l'une des revendications 2 à 12, **caractérisée en ce que** le polymère à bas point de fusion et/ou celui à haut point de fusion sont choisis parmi les copolymères résultant de la polymérisation d'au moins un monomère à chaîne cristallisable choisi parmi les (méth)acrylates d'alkyle saturés en C₁₄-C₂₄, les (méth)acrylates de perfluoroalkyle en C₁₁-C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ éventuellement fluoré.

14. Composition selon l'une des revendications 2 à 12, **caractérisée en ce que** les polymères semi-cristallins sont choisis parmi les homopolymères d'alkyl(méth)acrylate ou d'alkyl(méth)acrylamide avec un groupe alkyle en C₁₄ à C₂₄, les copolymères de ces monomères avec un monomère hydrophile et leurs mélanges.

15. Composition selon l'une des revendications 2 à 13, **caractérisée en ce que** les polymères semi-cristattins sont choisis parmi les copolymères d'alkyl(méth)acrylate ou d'alkyl (méth)acrylamide avec un groupe alkyle en C₁₄ à C₂₄ avec un monomère hydrophile de nature différente de l'acide (méth)acrylique comme la N-vinylpyrolidone ou l'hydroxyéthyl (méth)acrylate, et leurs mélanges.

16. Composition selon l'une des revendications 2 à 15, **caractérisée en ce que** le polymère à bas point de fusion et/ou celui à haut point de fusion sont issus d'un monomère à chaîne cristallisable choisi parmi les (méth)acrylates d'alkyle saturés en C₁₄ à C₂₂.

17. Composition selon l'une des revendications 2 à 16 **caractérisée en ce que** la chaîne organique cristallisable et/ou la séquence cristallisable du polymère à bas point de fusion et/ou de celui à haut point de fusion représentent au moins 30 % du poids total de chaque polymère.

18. Composition selon l'une des revendications 1 à 17, **caractérisée en ce que** le polymère à bas point de fusion et/ou le composé à haut point de fusion représentent de 0,1 à 80 % du poids total de la composition et mieux de 0,5 à 40 % et encore mieux plus de 10 %.

19. Composition selon l'une des revendications 1 à 18 **caractérisée en ce que** le composé à haut point de fusion et le polymère à bas point de fusion sont dans un rapport en poids allant de 90/10 à 10/90 et mieux de 40/60 à 60/40.

20. Composition selon l'une des revendications précédentes, **caractérisée en ce que** la phase grasse liquide représente de 5 à 99 % du poids total de la composition et mieux de 20 à 80 %.

21. Composition selon l'une des revendications précédentes, **caractérisée en ce que** la phase grasse contient au moins une huile hydrocarbonée d'origine minérale ou synthétique.

22. Composition selon l'une des revendications précédentes, **caractérisée en ce qu'**elle constitue une composition de soin et/ou de traitement et/ou de maquillage des matières kératiniques.

23. Composition selon l'une des revendications précédentes, **caractérisée en ce u'**elle contient, en outre, au moins une matière colorante.

24. Composition selon la revendication précédente, **caractérisée en ce que** la matière colorante est choisie parmi les colorants lipophiles, les colorants hydrophiles, les pigments, les nacres et leurs mélanges.

25. Composition selon la revendication 23 ou 24, **caractérisée en ce que** la matière colorante est présente à raison de 0,01 à 50 % du poids total de la composition, de préférence de 5 à 30 %.

26. Composition selon la revendication 24 à 25, **caractérisée en ce que** les pigments et les nacres sont introduits sous forme de pâte pigmentaire.

27. Composition selon l'une quelconque des revendications précédentes, **caractérisée en ce qu'**elle contient au moins un additif choisi parmi l'eau, les antioxydants, les huiles essentielles, les conservateurs, les neutralisants, les parfums, les cires, les corps gras pâteux et leurs mélanges.

28. Composition selon l'une des revendications précédentes, **caractérisée en ce qu'**elle contient moins de 10 %, en poids, de cire et/ou moins de 5 % en poids de charge matifiante, par rapport au poids total de la composition.

29. Composition selon l'une des revendications précédentes, **caractérisée en ce que** la phase grasse liquide représente la phase continue de la composition.

30. Composition selon l'une des revendications précédentes, **caractérisée en ce que** le rapport pondéral de polymère semi-cristallin à structure organique par rapport à la phase grasse liquide va de 0,20 à 0,50 et mieux de 0,25 à 0,45.

31. Composition selon l'une des revendications précédentes, **caractérisée en ce que** la composition est sous forme anhydre.

32. Composition selon l'une quelconque des revendications précédentes, **caractérisée en ce qu'**elle se présente sous forme coulée.

33. Composition selon l'une quelconque des revendications précédentes, **caractérisée en ce qu'**elle se présente sous forme de mascara, d'eye-liner, de fond de teint, de rouge à lèvres, de déodorant, de produit de maquillage du corps, de fard à paupières ou à joues, de produit anti-cerne.

34. Rouge à lèvres contenant au moins une phase grasse liquide structurée par au moins un polymère à bas point de fusion, semi-cristallin à structure organique, solide à température ambiante et ayant une température de fusion inférieure à 50°C, comportant a) un squelette polymérique et b) au moins une chaîne organique latérale cristallisable et/ ou une séquence organique cristallisable faisant partie dudit squelette polymérique du polymère à bas point de fusion, ledit polymère ayant une masse moléculaire moyenne en nombre supérieure à 2 000, la phase grasse liquide et le polymère formant un milieu physiologiquement acceptable pour les lèvres,
**caractérisé en ce qu'**il contient, en outre, un composé cristallin ou semi-cristallin à structure organique et à haut point de fusion, solide à température ambiante et ayant une température de fusion au moins égale à 50°C, et **en ce que** le polymère à bas point de fusion et/ou celui à haut point de fusion sont choisis parmi les homopolymères et copolymères résultant de la polymérisation d'au moins un monomère à chaîne(s) cristallisable(s) de formule X : 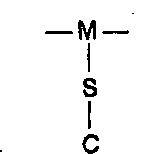 avec M représentant un atome du squelette polymérique
S représentant un espaceur
C représentant un groupe cristallisable
et leurs mélanges, avec « S-C » représentant une chaîne alkyle à au moins 11 atomes de carbone, éventuellement fluorée ou perfluorée.

35. Rouge à lèvres selon la revendication 34 **caractérisé en ce que** le composé cristallin ou semi-cristallin est un second polymère semi-cristallin comportant i) un squelette polymérique et ii) au moins une chaîne organique latérale cristallisable et/ou une séquence organique cristallisable faisant partie du squelette dudit second polymère, ledit second polymère ayant une masse moléculaire moyenne en nombre supérieure à 2 000.

36. Rouge à lèvres, **caractérisé en ce qu'**il se présente sous forme d'une composition conforme à l'une quelconque des revendications 1 à 32.

37. Procédé cosmétique de soin, de maquillage ou de traitement des matières kératiniques des êtres humains, comprenant l'application sur les matières kératiniques de la composition cosmétique conforme à l'une des revendications précédentes.

38. Utilisation cosmétique de l'association d'un premier polymère semi-cristallin à structure organique solide à température ambiante et ayant une température de fusion inférieure à 50°C, comportant a) un squelette polymérique et b) au moins une chaîne organique latérale cristallisable et/ou une séquence organique cristallisable faisant partie du squelette du premier polymère, ledit premier polymère ayant une masse moléculaire moyenne en nombre supérieure à 2 000, avec un second polymère semi-cristallin à structure organique et à haut point de fusion, solide à température ambiante et ayant une température de fusion au moins égale à 50°C, comportant i) un squelette polymérique et ii) au moins une chaîne organique latérale cristallisable et/ou une séquence organique cristallisable faisant partie du squelette du second polymère, ledit second polymère ayant une masse moléculaire moyenne en nombre supérieure à 2 000 ledit polymère à bas point de fusion et/ou celui à haut point de fusion étant choisis parmi les homopolymères et copolymères résultant de la polymérisation d'au moins un monomère à chaîne(s) cristallisable(s) de formule X : 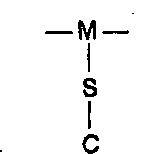 avec M représentant un atome du squelette polymérique
S représentant un espaceur
C représentant un groupe cristallisable
et leurs mélanges, avec « S-C » représentant une chaîne alkyle à au moins 11 atomes de carbone, éventuellement fluorée ou perfluorée, dans une composition notamment cosmétique contenant un milieu physiologiquement acceptable, brillante et/ou non migrante et/ou non collante et/ou couvrante.

39. Utilisation selon la revendication 38, dans laquelle le rapport pondéral de polymère semi-cristallin à structure organique par rapport à la phase grasse liquide va de 0,20 à 0,50 et mieux de 0,25 à 0,45.

## Claims

1. Composition containing at least one liquid fatty phase structured with at least one low-melting semi-crystalline polymer of organic structure, which is solid at room temperature and which has a melting point of less than 50°C, comprising a) a polymer backbone and b) at least one crystallizable organic side chain and/or one crystallizable organic block forming part of the polymer backbone of the said polymer, the said polymer having a number-average molecular mass of greater than 2000, the liquid fatty phase and the polymer forming a physiologically acceptable medium, **characterized in that** it also contains a high-melting crystalline or semi-crystalline compound of organic structure, which is solid at room temperature and which has a melting point at least equal to 50°C, and **in that** the low-melting polymer and/or the high-melting polymer are chosen from homopolymers and copolymers resulting from the polymerization of at least one monomer with crystallizable chain(s) of formula X: 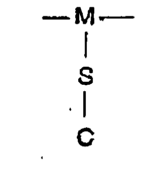 with M representing an atom of the polymer backbone,
S representing a spacer and
C representing a crystallizable group
and mixtures thereof, with "S-C" representing an optionally fluorinated or perfluorinated alkyl chain containing at least 11 carbon atoms.

2. Composition according to Claim 1, **characterized in that** the crystalline or semi-crystalline compound is a second high-melting polymer comprising i) a polymer backbone and ii) at least one crystallizable side chain and/or one crystallizable organic block forming part of the backbone of the said second polymer, the said second polymer having a number-average molecular mass of greater than 2000.

3. Composition according to Claim 2, **characterized in that** the low-melting polymer and/or the high-melting polymer have a number-average molecular mass ranging from 3000 to 500 000 and better still from 4000 to 99 000.

4. Composition according to any one of Claims 1 to 3, **characterized in that** the low-melting polymer and/or the high-melting polymer are soluble in the liquid fatty phase at a temperature above their melting point.

5. Composition according to one of the preceding claims, **characterized in that** the low-melting polymer has a melting point Tm₂ such that 30°C ≤ Tm₂ < 50°C.

6. Composition according to one of Claims 1 to 5, **characterized in that** the high-melting compound has a melting point Tm₁ such that 55°C ≤ Tm₁ ≤ 150°C and preferably 60°C ≤ Tm₁ ≤ 130°C.

7. Composition according to one of Claims 2 to 6, **characterized in that** the low-melting polymer and/or the high-melting polymer are chosen from block copolymers comprising at least one crystallizable block and at least one amorphous block, the homopolymers and the copolymers bearing at least one crystallizable side chain per repeating unit, and mixtures thereof.

8. Composition according to Claim 7, **characterized in that** the crystallizable block is of different nature from the amorphous block.

9. Composition according to one of Claims 2 to 7, **characterized in that** the low-melting polymer and/or the high-melting polymer are chosen from:
- block copolymers of controlled-crystallization polyolefins,
- aliphatic or aromatic polyester polycondensates and aliphatic/aromatic copolyesters,
- homopolymers or copolymers bearing at least one crystallizable side chain,
- mixtures thereof.

10. Composition according to one of Claims 2 to 9, **characterized in that** the low-melting polymer and/or the high-melting polymer are chosen from homopolymers and copolymers comprising from 50% to 100% by weight of units resulting from the polymerization of one or more monomers bearing crystallizable hydrophobic side chain(s).

11. Composition according to one of Claims 2 to 10, **characterized in that** the low-melting polymer and/or the high-melting polymer are chosen from polymers resulting from the polymerization of at least one monomer chosen from acrylic acid, methacrylic acid, crotonic acid, itaconic acid, maleic acid and maleic anhydride, and mixtures thereof.

12. Composition according to one of Claims 2 to 11, **characterized in that** the low-melting polymer and/or the high-melting polymer are chosen from homopolymers and copolymers resulting from the polymerization of at least one monomer with a crystallizable chain chosen from saturated C₁₄-C₂₄ alkyl (meth) acrylates, C₁₁-C₁₅ perfluoroalkyl (meth)acrylates, C₁₄-C₂₄ N-alkyl (meth) - acrylamides with or without a fluorine atom, vinyl esters containing C₁₄-C₂₄ alkyl or perfluoroalkyl chains, vinyl ethers containing C₁₄-C₂₄ alkyl or perfluoroalkyl chains, C₁₄-C₂₄ α-olefins, and para-alkylstyrenes with an alkyl group containing from 12 to 24 carbon atoms, and mixtures thereof.

13. Composition according to one of Claims 2 to 12, **characterized in that** the low-melting polymer and/or the high-melting polymer are chosen from copolymers resulting from the polymerization of at least one monomer with a crystallizable chain chosen from saturated C₁₄-C₂₄ alkyl (meth) acrylates, C₁₁-C₁₅ perfluoroalkyl (meth) acrylates, C₁₄-C₂₄ N-alkyl(meth)-acrylamides with or without a fluorine atom, vinyl esters containing C₁₄-C₂₄ alkyl or perfluoroalkyl chains, vinyl ethers containing C₁₄-C₂₄ alkyl or perfluoroalkyl chains, C₁₄-C₂₄ α-olefins, and para-alkylstyrenes with an alkyl group containing from 12 to 24 carbon atoms, with at least one optionally fluorinated C₁-C₁₀ monocarboxylic acid ester or amide.

14. Composition according to one of Claims 2 to 12, **characterized in that** the semi-crystalline polymers are chosen from alkyl (meth)acrylate or alkyl(meth)-acrylamide homopolymers with a C₁₄-C₂₄ alkyl group, and copolymers of these monomers with a hydrophilic monomer, and mixtures thereof.

15. Composition according to one of Claims 2 to 13, **characterized in that** the semi-crystalline polymers are chosen from alkyl (meth) acrylate or alkyl(meth)-acrylamide copolymers with a C₁₄-C₂₄ alkyl group with a hydrophilic monomer of different nature from (meth)acrylic acid, for instance N-vinylpyrrolidone or hydroxyethyl (meth)acrylate, and mixtures thereof.

16. Composition according to one of Claims 2 to 15, **characterized in that** the low-melting polymer and/or the high-melting polymer are derived from a monomer with a crystallizable chain chosen from saturated C₁₄-C₂₂ alkyl (meth)acrylates.

17. Composition according to any one of Claims 2 to 16, **characterized in that** the crystallizable organic chain and/or the crystallizable block of the low-melting polymer and/or of the high-melting polymer represent at least 30% of the total weight of each polymer.

18. Composition according to one of Claims 1 to 17, **characterized in that** the low-melting polymer and/or the high-melting compound represent from 0.1% to 80%, better still from 0.5% to 40% and even better still more than 10% of the total weight of the composition.

19. Composition according to one of Claims 1 to 18, **characterized in that** the high-melting compound and the low-melting polymer are in a weight ratio ranging from 90/10 to 10/90 and better still from 40/60 to 60/40.

20. Composition according to one of the preceding claims, **characterized in that** the liquid fatty phase represents from 5% to 99% and better still from 20% to 80% of the total weight of the composition.

21. Composition according to one of the preceding claims, **characterized in that** the fatty phase contains at least one hydrocarbon-based oil of mineral or synthetic origin.

22. Composition according to one of the preceding claims, **characterized in that** it constitutes a care and/or treatment and/or makeup composition for keratin materials.

23. Composition according to one of the preceding claims, **characterized in that** it also contains at least one dyestuff.

24. Composition according to the preceding claim, **characterized in that** the dyestuff is chosen from lipophilic dyes, hydrophilic dyes, pigments and nacres, and mixtures thereof.

25. Composition according to Claim 23 or 24, **characterized in that** the dyestuff is present in a proportion of from 0.01% to 50% and preferably from 5% to 30% of the total weight of the composition.

26. Composition according to Claim 24 or 25, **characterized in that** the pigments and nacres are introduced in the form of a pigmentary paste.

27. Composition according to any one of the preceding claims, **characterized in that** it contains at least one additive chosen from water, antioxidants, essential oils, preserving agents, neutralizers, fragrances, waxes and pasty fatty substances, and mixtures thereof.

28. Composition according to one of the preceding claims, **characterized in that** it contains less than 10% by weight of wax and/or less than 5% by weight of matting filler, relative to the total weight of the composition.

29. Composition according to one of the preceding claims, **characterized in that** the liquid fatty phase represents the continuous phase of the composition.

30. Composition according to one of the preceding claims, **characterized in that** the weight ratio of semi-crystalline polymer of organic structure relative to the liquid fatty phase ranges from 0.20 to 0.50 and better still from 0.25 to 0.45.

31. Composition according to one of the preceding claims, **characterized in that** the composition is in anhydrous form.

32. Composition according to any one of the preceding claims, **characterized in that** it is in cast form.

33. Composition according to any one of the preceding claims, **characterized in that** it is in the form of a mascara, an eyeliner, a foundation, a lipstick, a deodorant, a body makeup product, an eyeshadow, a makeup rouge or a concealer product.

34. Lipstick containing at least one liquid fatty phase structured with at least one low-melting semi-crystalline polymer of organic structure, which is solid at room temperature and which has a melting point of less than 50°C, comprising a) a polymer backbone and b) at least one crystallizable organic side chain and/or one crystallizable organic block forming part of the said polymer backbone of the low-melting polymer, the said polymer having a number-average molecular mass of greater than 2000, the liquid fatty phase and the polymer forming a medium that is physiologically acceptable for the lips, **characterized in that** it also contains a high-melting crystalline or semi-crystalline compound of organic structure, which is solid at room temperature and which has a melting point at least equal to 50°C, and **in that** the low-melting polymer and/or the high-melting polymer are chosen from homopolymers and copolymers resulting from the polymerization of at least one monomer with crystallizable chain(s) of formula X: 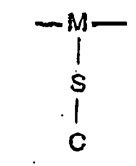 with M representing an atom of the polymer backbone,
S representing a spacer and
C representing a crystallizable group
and mixtures thereof, with "S-C" representing an optionally fluorinated or perfluorinated alkyl chain containing at least 11 carbon atoms.

35. Lipstick according to Claim 34, **characterized in that** the crystalline or semi-crystalline compound is a second semi-crystalline polymer comprising i) a polymer backbone and ii) at least one crystallizable organic side chain and/or one crystallizable organic block forming part of the backbone of the said second polymer, the said second polymer having a number-average molecular mass of greater than 2000.

36. Lipstick, **characterized in that** it is in the form of a composition in accordance with any one of Claims 1 to 32.

37. Cosmetic process for caring for, making up or treating human keratin materials, comprising the application to the keratin materials of the cosmetic composition in accordance with one of the preceding claims.

38. Cosmetic use of the combination of a first semi-crystalline polymer of organic structure, which is solid at room temperature and which has a melting point of less than 50°C, comprising a) a polymer backbone and b) at least one crystallizable organic side chain and/or one crystallizable organic block forming part of the backbone of the first polymer, the said first polymer having a number-average molecular mass of greater than 2000, with a second high-melting semi-crystalline polymer of organic structure, which is solid at room temperature and which has a melting point at least equal to 50°C, comprising i) a polymer backbone and ii) at least one crystallizable organic side chain and/or one crystallizable organic block forming part of the backbone of the second polymer, the said second polymer having a number-average molecular mass of greater than 2000, the said low-melting polymer and/or the said high-melting polymer being chosen from homopolymers and copolymers resulting from the polymerization of at least one monomer with crystallizable chain(s) of formula X: 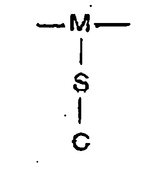 with M representing an atom of the polymer backbone,
S representing a spacer and
C representing a crystallizable group
and mixtures thereof, with "S-C" representing an optionally fluorinated or perfluorinated alkyl chain containing at least 11 carbon atoms, in a glossy and/or migration-resistant and/or non-tacky and/or covering composition, especially a cosmetic composition, containing a physiologically acceptable medium.

39. Use according to Claim 38, in which the weight ratio of semi-crystalline polymer of organic structure relative to the liquid fatty phase ranges from 0.20 to 0.50 and better still from 0.25 to 0.45.

## Patentansprüche

1. Zusammensetzung, die mindestens eine flüssige Fettphase enthält, die mit mindestens einem teilkristallinen Polymer mit niedrigem Schmelzpunkt und organischer Struktur strukturiert ist, das bei Raumtemperatur fest ist, einen Schmelzpunkt unter 50 °C aufweist und a) ein Polymergrundgerüst und b) mindestens eine kristallisierbare organische Seitenkette und/oder eine kristallisierbare organische Sequenz, die Teil des Polymergrundgerüsts des Polymers ist, besitzt, wobei das Polymer eine zahlenmittlere Molmasse über 2 000 aufweist und wobei die flüssige Fettphase und das Polymer ein physiologisch akzeptables Medium bilden, **dadurch gekennzeichnet, dass** sie ferner eine kristalline oder teilkristalline Verbindung mit organischer Struktur und mit hohem Schmelzpunkt enthält, die bei Umgebungstemperatur fest ist und eine Schmelztemperatur von mindestens 50 °C aufweist, und **dadurch**, dass das Polymer mit niedrigem Schmelzpunkt und/oder das Polymer mit hohem Schmelzpunkt unter den Homopolymeren und Copolymeren, die bei der Polymerisation mindestens eines Monomers mit kristallisierbarer Kette oder kristallisierbaren Ketten der Formel X gebildet werden: 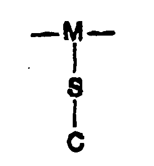 wobei M ein Atom des Polymergrundgerüsts bedeutet, S eine Spacergruppe ist und C eine kristallisierbare Gruppe bedeutet, und deren Gemischen ausgewählt sind, wobei «S-C» eine Alkylkette mit mindestens 11. Kohlenstoffatomen bedeutet, die gegebenenfalls fluoriert oder perfluoriert ist.

2. Zusammensetzung nach Anspruch 1, **dadurch gekennzeichnet, dass** die kristalline oder halbkristalline Verbindung ein zweites Polymer mit hohem Schmelzpunkt ist, das i) ein Polymergrundgerüst und ii) mindestens eine kristallisierbare Seitenkette und/oder eine kristallisierbare organische Sequenz, die Teil des Grundgerüsts des zweiten Polymers ist, aufweist, wobei das zweite Polymer eine zahlenmittlere Molmasse über 2 000 besitzt.

3. Zusammensetzung nach Anspruch 2, **dadurch gekennzeichnet, dass** das Polymer mit niedrigem Schmelzpunkt und/ oder das Polymer mit hohem Schmelzpunkt eine zahlenmittlere Molmasse von 3 000 bis 500 000 und besser 4 000 bis 99 000 aufweisen.

4. Zusammensetzung nach einem der Ansprüche 1 bis 3, **dadurch gekennzeichnet, dass** das Polymer mit niedrigem Schmelzpunkt und/oder die Verbindung mit hohem Schmelzpunkt in der flüssigen Fettphase bei einer Temperatur über ihrer Schmelztemperatur löslich sind.

5. Zusammensetzung nach einem der vorhergehenden Ansprüche, **dadurch gekennzeichnet, dass** das Polymer mit niedrigem Schmelzpunkt eine Schmelztemperatur Fₚ₂ aufweist, die so ist, dass 30 °C ≤ Fₚ₂ 50 °C.

6. Zusammensetzung nach einem der Ansprüche 1 bis 5, **dadurch gekennzeichnet, dass** die Verbindung mit hohem Schmelzpunkt eine Schmelztemperatur Fₚ₁ aufweist, die so ist, dass 55 °C≤ Fₚ₁ < 150 °C und vorzugsweise 60 °C ≤ Fₚ₁≤ 130 °C.

7. Zusammensetzung nach einem der Ansprüche 2 bis 6, **dadurch gekennzeichnet, dass** das Polymer mit niedrigem Schmelzpunkt und/oder das Polymer mit hohem Schmelzpunkt unter den Sequenzcopolymeren, die mindestens eine kristallisierbare Sequenz und mindestens eine amorphe Sequenz aufweisen, und deren Gemischen ausgewählt sind, wobei die Homopolymere und Copolymere mindestens eine kristallisierbare Seitenkette pro Wiederholungseinheit aufweisen.

8. Zusammensetzung nach Anspruch 7, **dadurch gekennzeichnet, dass** die kristallisierbare Sequenz in ihrer Art von der amorphen Sequenz verschieden ist.

9. Zusammensetzung nach einem der Ansprüche 2 bis 7, **dadurch gekennzeichnet, dass** das Polymer mit niedrigem Schmelzpunkt und/oder das Polymer mit hohem Schmelzpunkt ausgewählt sind unter:
- Sequenzcopolymeren von Polyolefinen mit kontrollierter Kristallisation,
- aliphatischen oder aromatischen Polyesterpolykondensaten und aliphatischen/aromatischen Copolyestern,
- Homo- oder Copolymeren, die mindestens eine kristallisierbare Seitenkette aufweisen, und
- deren Gemischen.

10. Zusammensetzung nach einem der Ansprüche 2 bis 9, **dadurch gekennzeichnet, dass** das Polymer mit niedrigem Schmelzpunkt und/oder das Polymer mit hohem Schmelzpunkt unter den Homopolymeren und Copolymeren ausgewählt sind, die 50 bis 100 Gew.-% Einheiten enthalten, die bei der Polymerisation eines oder mehrerer Monomere gebildet werden, die eine oder mehrere hydrophobe kristallisierbare Seitenketten aufweisen.

11. Zusammensetzung nach einem der Ansprüche 2 bis 10, **dadurch gekennzeichnet, dass** das Polymer mit niedrigem Schmelzpunkt und/oder das Polymer mit hohem Schmelzpunkt unter den Polymeren ausgewählt sind, die bei der Polymerisation mindestens eines Monomers gebildet werden, das unter Acrylsäure, Methacrylsäure, Crotonsäure, Itaconsäure, Maleinsäure, Maleinsäureanhydrid und deren Gemischen ausgewählt ist.

12. Zusammensetzung nach einem der Ansprüche 2 bis 11, **dadurch gekennzeichnet, dass** das Polymer mit niedrigem Schmelzpunkt und/oder das Polymer mit hohem Schmelzpunkt unter den Homopolymeren und Copolymeren ausgewählt sind, die bei der Polymerisation mindestens eines Monomers mit kristallisierbarer Kette gebildet werden, das unter den gesättigten C₁₄₋₂₄-Alkyl-(meth)acrylaten, C₁₁₋₁₅-Perfluoralkyl(meth)acrylaten, N-Alkyl-(meth)acrylamiden mit 14 bis 24 Kohlenstoffatomen und mit oder ohne Fluoratom, Vinylestern mit Alkyl- oder Perfluoralkylketten mit 14 bis 24 Kohlenstoffatomen, Vinylethern mit Alkyl- oder Perfluoralkylketten mit 14 bis 24 Kohlenstoffatomen, C₁₄₋₂₄-alpha-Olefinen, para-Alkylstyrolen mit einer Alkylgruppe, die 12 bis 24 Kohlenstoffatome besitzt, und deren Gemischen ausgewählt ist.

13. Zusammensetzung nach einem der Ansprüche 2 bis 12, **dadurch gekennzeichnet, dass** das Polymer mit niedrigem Schmelzpunkt und/oder das Polymer mit hohem Schmelzpunkt unter den Copolymeren ausgewählt sind, die bei der Polymerisation mindestens eines Monomers mit kristallisierbarer Kette, das unter den gesättigten C₁₄₋₂₄-Alkyl(meth)acrylaten, C₁₁₋₁₅- Perfluoralkyl-(meth)acrylaten, N-Alkyl(meth)acrylamiden mit 14 bis 24 Kohlenstoffatomen und mit oder ohne Fluoratom, Vinylestern mit Alkyl- oder Perfluoralkylketten mit 14 bis 24 Kohlenstoffatomen, Vinylethern mit Alkyl- oder Perfluoralkylketten mit 14 bis 24 Kohlenstoffatomen, C₁₄₋₂₄-alpha-Olefinen, para-Alkylstyrolen mit einer Alkylgruppe, die 12 bis 24 Kohlenstoffatome aufweist, ausgewählt ist, und mindestens eines Esters oder Amids einer gegebenenfalls fluorierten C₁₋₁₀-Monocarbonsäure gebildet werden.

14. Zusammensetzung nach einem der Ansprüche 2 bis 12, **dadurch gekennzeichnet, dass** die halbkristallinen Polymere unter den Homopolymeren von Alkyl(meth)acrylat oder Alkyl(meth)acrylamid mit einer C₁₄₋₂₄-Alkylgruppe, den Copolymeren dieser Monomere mit einem hydrophilen Monomer und deren Gemischen ausgewählt sind.

15. Zusammensetzung nach einem der Ansprüche 2 bis 13, **dadurch gekennzeichnet, dass** die halbkristallinen Polymere unter den Copolymeren von Alkyl(meth)acrylat oder Alkyl(meth)acrylamid mit C₁₄₋₂₄-Alkylgruppe und einem hydrophilen Monomer, das in seiner Art von (Meth)acrylsäure verschieden ist, wie N-Vinylpyrrolidon oder Hydroxyethyl(meth)acrylat, und deren Gemischen ausgewählt sind.

16. Zusammensetzung nach einem der Ansprüche 2 bis 15, **dadurch gekennzeichnet, dass** das Polymer mit niedrigem Schmelzpunkt und/oder das Polymer mit hohem Schmelzpunkt von einem Monomer mit kristallisierbarer Kette abgeleitet sind, das unter den gesättigten C₁₄₋₂₂-Alkyl(meth)acrylaten ausgewählt ist.

17. Zusammensetzung nach einem der Ansprüche 2 bis 16, **dadurch gekennzeichnet, dass** die kristallisierbare organische Kette und/oder kristallisierbare Sequenz des Polymers mit niedrigem Schmelzpunkt und/oder des Polymers mit hohem Schmelzpunkt mindestens 30 % des Gesamtgewichts jedes Polymers ausmachen.

18. Zusammensetzung nach einem der Ansprüche 1 bis 17, **dadurch gekennzeichnet, dass** das Polymer mit niedrigem Schmelzpunkt und/oder die Verbindung mit hohem Schmelzpunkt 0,1 bis 80 Gew.-% des Gesamtgewichts der Zusammensetzung, besser 0,5 bis 40 % und noch besser mehr als 10 % ausmachen.

19. Zusammensetzung nach einem der Ansprüche 1 bis 18, **dadurch gekennzeichnet, dass** die Verbindung mit hohem Schmelzpunkt und das Polymer mit niedrigem Schmelzpunkt in einem Gewichtsverhältnis von 90/10 bis 10/90 und besser 40/60 bis 60/40 enthalten sind.

20. Zusammensetzung nach einem der vorhergehenden Ansprüche, **dadurch gekennzeichnet, dass** die flüssige Fettphase 5 bis 99 % des Gesamtgewichts der Zusammensetzung und besser 20 bis 80 % ausmacht.

21. Zusammensetzung nach einem der vorhergehenden Ansprüche, **dadurch gekennzeichnet, dass** die Fettphase mindestens ein Kohlenwasserstofföl mineralischer oder synthetischer Herkunft enthält.

22. Zusammensetzung nach einem der vorhergehenden Ansprüche, **dadurch gekennzeichnet, dass** es sich um eine Zusammensetzung für die Pflege und/oder für die Behandlung und/oder zum Schminken von Keratinsubstanzen handelt.

23. Zusammensetzung nach einem der vorhergehenden Ansprüche, **dadurch gekennzeichnet, dass** sie ferner mindestens ein Farbmittel enthält.

24. Zusammensetzung nach dem vorhergehenden Anspruch, **dadurch gekennzeichnet, dass** das Farbmittel unter den lipophilen Farbstoffen, hydrophilen Farbstoffen, Pigmenten, Perlglanzpigmenten und deren Gemischen ausgewählt ist.

25. Zusammensetzung nach Anspruch 23 oder 24, **dadurch gekennzeichnet, dass** das Farbmittel in einer Menge von 0,01 bis 50 % des Gesamtgewichts der Zusammensetzung und vorzugsweise 5 bis 30 % enthalten ist.

26. Zusammensetzung nach Anspruch 24 bis 25, **dadurch gekennzeichnet, dass** die Pigmente und Perlglanzpigmente in Form einer Pigmentpaste eingebracht werden.

27. Zusammensetzung nach einem der vorhergehenden Ansprüche, **dadurch gekennzeichnet, dass** sie mindestens einen Zusatzstoff enthält, der unter Wasser, Antioxidantien, etherischen Ölen, Konservierungsmitteln, Neutralisationsmitteln, Parfums, Wachsen, pastösen Fettsubstanzen und deren Gemischen ausgewählt ist.

28. Zusammensetzung nach einem der vorhergehenden Ansprüche, **dadurch gekennzeichnet, dass** sie weniger als 10 Gew.-% Wachs und/oder weniger als 5 Gew.-% mattierenden Füllstoff, bezogen auf das Gesamtgewicht der Zusammensetzung, enthält.

29. Zusammensetzung nach einem der vorhergehenden Ansprüche, **dadurch gekennzeichnet, dass** die flüssige Fettphase die kontinuierliche Phase der Zusammensetzung bildet.

30. Zusammensetzung nach einem der vorhergehenden Ansprüche, **dadurch gekennzeichnet, dass** das Gewichtsverhältnis des halbkristallinen Polymers mit organischer Struktur bezogen auf die flüssige Fettphase im Bereich von 0,20 bis 0,50 und besser 0,25 bis 0,45 liegt.

31. Zusammensetzung nach einem der vorhergehenden Ansprüche, **dadurch gekennzeichnet, dass** die Zusammensetzung in wasserfreier Form vorliegt.

32. Zusammensetzung nach einem der vorhergehenden Ansprüche, **dadurch gekennzeichnet, dass** sie in gegossener Form vorliegt.

33. Zusammensetzung nach einem der vorhergehenden Ansprüche, **dadurch gekennzeichnet, dass** sie als Mascara, Eyeliner, Makeup, Lippenrot, Deodorant, Schminkprodukt für den Körper, Lidschatten, Wangenrouge oder Produkt gegen Augenringe vorliegt.

34. Lippenstift, der mindestens eine flüssige Fettphase enthält, die mit mindestens einem halbkristallinen Polymer mit niedrigem Schmelzpunkt und organischer Struktur strukturiert ist, das bei Raumtemperatur fest ist, eine Schmelztemperatur unter 50 °C aufweist und a) ein Polymergrundgerüst und b) mindestens eine kristallisierbare organische Seitenkette und/oder eine kristallisierbare organische Sequenz aufweist, die Teil des Polymergrundgerüsts des Polymers mit niedrigem Schmelzpunkt ist, wobei das Polymer eine zahlenmittlere Molmasse über 2 000 auf weist und wobei die flüssige Fettphase und das Polymer ein für die Lippen physiologisch verträgliches Medium bilden, **dadurch gekennzeichnet, dass** er ferner eine kristalline oder halbkristalline Verbindung mit organischer Struktur und hohem Schmelzpunkt enthält, die bei Raumtemperatur fest ist und eine Schmelztemperatur von mindestens 50 °C aufweist, und **dadurch**, dass das Polymer mit niedrigem Schmelzpunkt und/oder das Polymer mit hohem Schmelzpunkt unter den Homopolymeren und Copolymeren, die bei der Polymerisation mindestens eines Monomers mit kristallisierbarer Kette oder kristallisierbaren Ketten der Formel X gebildet werden: 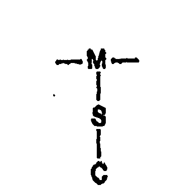 wobei M ein Atom eines Polymergrundgerüsts bedeutet, S eine Spacergruppe ist und C eine kristallisierbare Gruppe bedeutet, und deren Gemischen ausgewählt sind, wobei «S-C» eine Alkylkette mit mindestens 11 Kohlenstoffatomen bedeutet, die gegebenenfalls fluoriert oder perfluoriert ist.

35. Lippenstift nach Anspruch 34, **dadurch gekennzeichnet, dass** die kristalline oder halbkristalline Verbindung ein zweites halbkristallines Polymer ist, das i) ein Polymergrundgerüst und ii) mindestens eine kristallisierbar organische Seitenkette und/oder eine kristallisierbare organische Sequenz aufweist, die Teil des Grundgerüsts des zweiten Polymers ist, wobei das zweite Polymer eine zahlenmittlere Molmasse über 2 000 aufweist.

36. Lippenstift, **dadurch gekennzeichnet, dass** er in Form einer Zusammensetzung nach einem der Ansprüche 1 bis 32 vorliegt.

37. Kosmetisches Verfahren für die Pflege, zum Schminken oder für die Behandlung von menschlichen Keratinsubstanzen, das das Auftragen der kosmetischen Zusammensetzung nach einem der vorhergehenden Ansprüche auf die Keratinsubstanzen umfasst.

38. Kosmetische Verwendung der Kombination eines ersten halbkristallinen Polymers mit organischer Struktur, das bei Umgebungstemperatur fest ist, eine Schmelztemperatur unter 50 °C aufweist und a) ein Polymergrundgerüst und b) mindestens eine kristallisierbare organische Seitenkette und/oder eine kristallisierbare organische Sequenz aufweist, die Teil des Grundgerüsts des ersten Polymers ist, wobei das erste Polymer eine zahlenmittlere Molmasse über 2 000 besitzt, und eines zweiten halbkristallinen Polymers mit organischer Struktur und hohem Schmelzpunkt, das bei Umgebungstemperatur fest ist und einen Schmelzpunkt von mindestens 50 °C aufweist und i) ein Polymergrundgerüst und ii) mindestens eine kristallisierbare organische Seitenkette und/oder eine kristallisierbare organische Sequenz besitzt, die Teil des Grundgerüsts des zweiten Polymers ist, wobei das zweite Polymer eine zahlenmittlere Molmasse über 2 000 aufweist und wobei das Polymer mit niedrigem Schmelzpunkt und/ oder das Polymer mit hohem Schmelzpunkt unter den Homopolymeren und Copolymeren, die bei der Polymerisation mindestens eines Monomers mit kristallisierbarer Kette oder kristallisierbaren Ketten der Formel X gebildet werden: 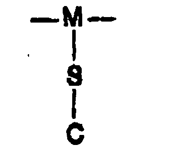 wobei M ein Atom eines Polymergrundgerüsts bedeutet, S eine Spacergruppe ist und C eine kristallisierbare Gruppe bedeutet, und deren Gemischen ausgewählt sind, wobei «S-C» eine Alkylkette mit mindestens 11 Kohlenstoffatomen bedeutet, die gegebenenfalls fluoriert oder perfluoriert ist, in einer Zusammensetzung und insbesondere in einer kosmetischen Zusammensetzung, die ein physiologisch akzeptables Medium enthält und die glänzt und/oder keine Migration zeigt und/oder nicht färbt und/oder abdeckt.

39. Verwendung nach Anspruch 38, wobei das Gewichtsverhältnis des halbkristallinen Polymers mit organischer Struktur und der flüssigen Fettphase im Bereich von 0,20 bis 0,50 und besser 0,25 bis 0,45 liegt.
